# FASCICULE DE BREVET EUROPEEN

(11) **EP 0 703 204 B1**
(45) Date de publication et mention de la délivrance du brevet: **09.06.1999**
(21) Numéro de dépôt: 95202518.7
(22) Date de dépôt: 18.09.1995
(51) Int. Cl.: C07C 17/087, C07C 19/08

(54) **Procédé de préparation du 1,1,1-trifluoréthane**
Verfahren zur Herstellung von 1,1,1-Trifluorethan
Process for the preparation of 1,1,1-trifluoroethane

(30) Priorité: 26.09.1994 FR 9411563
(43) Date de publication de la demande: 27.03.1996
(73) Titulaire: SOLVAY (Société Anonyme), B-1050 Bruxelles (BE)
(72) Inventeur: Pennetreau, Pascal, B-1330 Rixensart (BE); Balthasart, Dominique, B-1120 Bruxelles (BE)
(74) Mandataire: Jacques, Philippe

(56) Documents cités:
- EP-A- 0 574 077
- WO-A-91/18852
- FR-A- 2 124 239

## Description

L'invention concerne un procédé pour la préparation du 1,1,1-trifluoroéthane (HFC-143a), par réaction entre du fluorure de vinylidène et du fluorure d'hydrogène en phase liquide.

Il est connu, notamment par le brevet US-2669590, de préparer du 1,1,1-trifluoroéthane par réaction de fluorure de vinylidène avec du fluorure d'hydrogène en phase gazeuse, en présence d'un catalyseur. La productivité d'un tel procédé par unité volumique de réacteur est cependant très faible.

Il est par ailleurs connu de préparer du 1,1,1-trifluoroéthane au départ des mêmes réactifs, en phase liquide, à une température de -50 °C, en présence de pentafluorure d'antimoine comme catalyseur (OLAH G.A. et MO Y.K.; Journal of Organic Chemistry, (1972), Vol. 37, No. 7). Dans un tel procédé, les sous-produits lourds formés en faible quantité sont contaminés par les métaux lourds utilisés comme catalyseurs. En outre, le pentafluorure d'antimoine est transformé progressivement en trifluorure d'antimoine particulièrement corrosif à l'encontre des matériaux métalliques.

La présente invention vise dès lors à fournir un procédé de préparation du 1,1,1-trifluoroéthane qui ne présente plus les inconvénients des procédés mentionnés ci-dessus et qui puisse être facilement mis en oeuvre industriellement.

L'invention concerne dès lors un procédé pour la préparation du 1,1,1-trifluoroéthane par réaction entre du fluorure de vinylidène et du fluorure d'hydrogène en phase liquide, qui se caractérise en ce qu'on réalise la réaction en absence de catalyseur. Par catalyseur, on entend tout composé qui augmente de manière substantielle la vitesse de réaction.

Il a en effet été observé de manière surprenante que le fluorure de vinylidène et le fluorure d'hydrogène réagissent très rapidement en phase liquide en absence de tout catalyseur pour former de manière très sélective du 1,1,1-tritluoroéthane, alors qu'en phase gazeuse, ils réagissent de manière très lente en absence de catalyseur.

Dans le procédé selon l'invention, le fluorure de vinylidène et le fluorure d'hydrogène peuvent être mis en oeuvre dans des rapports molaires variables. Généralement, on met en oeuvre au moins 1 mole de fluorure d'hydrogène par mole de fluorure de vinylidène. De préférence, ce rapport est d'au moins environ 2. Le plus souvent, on ne dépasse pas environ 20 moles de fluorure d'hydrogène par mole de fluorure de vinylidène. De manière avantageuse, ce rapport ne dépasse pas 10.

Le procédé selon l'invention peut être réalisé dans une large gamme de températures. Généralement, le procédé est mené à une température d'au moins environ -50 °C. De préférence, elle est d'au moins -30 °C. De bons résultats ont été obtenus à une température supérieure ou égale à -20 °C. Le plus souvent, la température de réaction ne dépasse pas environ 120 °C. De manière avantageuse, elle ne dépasse pas 100 °C. De bons résultats ont été obtenus à une température ne dépassant pas 90 °C.

La pression à laquelle est mené le procédé selon l'invention n'est pas critique en elle-même, dès lors qu'elle permet d'effectuer la réaction en phase liquide, c'est-à-dire qu'elle est suffisante pour maintenir les réactifs présents dans le réacteur essentiellement sous forme liquide. Elle varie en fonction de la température du mélange réactionnel. Cette pression peut être la pression autogène, une pression supérieure générée par l'introduction d'un gaz inerte, tel que par exemple l'azote, ou une pression inférieure obtenue par dilution du mélange réactionnel avec un solvant organique tel que, par exemple, le 1,2-dichloroéthane, le 1,1-dichloro-1-fluoroéthane, un chlorofluorobutane de formule brute C₄H₅Cl₅₋ₓFₓ, avec x un nombre de 0 à 5 ou un mélange de ces composés. Généralement, la réaction est effectuée à une pression au moins égale à 2 bar, de préférence au moins égale à 3 bar. Le plus souvent, la pression ne dépasse pas 30 bar. De manière avantageuse, elle ne dépasse pas 20 bar.

Le procédé selon l'invention peut être mis en oeuvre de manière discontinue mais est avantageusement effectué de manière continue.

Le temps de séjour des réactifs dans le réacteur, c'est-à-dire, en mode discontinu, la durée de réaction et, en mode continu, le rapport entre le volume de mélange réactionnel contenu dans le réacteur et le débit total des réactifs à l'état liquide, est généralement d'au moins environ 2 minutes. De préférence, il est d'au moins environ 5 minutes. Le plus souvent, il ne dépasse pas environ 2 heures. Un temps de séjour ne dépassant pas environ 1 heure est particulièrement recommandé.

Le procédé selon l'invention peut être réalisé dans tout réacteur réalisé dans un matériau résistant à la température et à la pression de travail et résistant au fluorure d'hydrogène dans les conditions de mise en oeuvre du procédé. On utilise avantageusement des réacteurs réalisés en acier au carbone, en acier inoxydable ou en alliages tels que ceux connus sous les marques MONEL, INCONEL ou HASTELLOY. On peut également utiliser des réacteurs munis d'un revêtement en un métal ou un alliage résistant au fluorure d'hydrogène, ou recouverts d'une couche d'une résine inerte dans les conditions de réaction, notamment une résine fluorée.

Le procédé selon l'invention présente l'avantage appréciable d'une productivité en 1,1,1-trifluoroéthane largement supérieure à celle atteinte par un procédé similaire réalisé en phase gazeuse. Il permet en outre d'obtenir une excellente sélectivité en 1,1,1-trifluoroéthane, la formation de sous-produits lourds étant très limitée. Il évite en outre la formation de boues résultant de la présence d'un catalyseur. De plus, en l'absence de catalyseurs, les sous-produits lourds éventuellement formés ne sont pas contaminés par des métaux lourds et peuvent dès lors être plus aisément détruits.

Les exemples suivants illustrent l'invention de manière non limitative.

### Exemple 1 (Comparaison) Hydrofluoration en phase gazeuse.

Dans un réacteur en acier inoxydable, ne renfermant aucun solide catalytique, on a introduit, sous forme gazeuse, du fluorure d'hydrogène (HF) et du fluorure de vinylidène (VF2) dans un rapport molaire HF/VF2 égal à 3. Le réacteur a été maintenu à une température de 135 °C et le temps de séjour des réactifs a été de 13 secondes. Dans ces conditions, la conversion du VF2 en 1,1,1-trifluoroéthane (HFC-143a) a été d'environ 7 %, soit une productivité moyenne en HFC-143a de 12 g.1⁻¹.h⁻¹.

### Exemple 2

Dans un autoclave en acier inoxydable de 0,5 l, équipé d'un agitateur mécanique, d'une sonde de température et d'un tube plongeant pour effectuer des prélèvements en phase liquide, préalablement mis sous vide et refroidi à environ -60 °C, on a introduit 77,8 g de HF, puis 20 g de VF2. Au terme de l'introduction du VF2, qui a duré 5 minutes, on a mesuré une température de -50 °C et une pression de 3 bar.

15 minutes après la fin de l'introduction du VF2, un prélèvement de la phase liquide a été effectué. La température était alors de -16 °C. L'analyse de ce prélèvement a révélé que la conversion du VF2 était déjà supérieure à 99.8 % et que tout le VF2 a été converti en HFC-143a (sélectivité = 100 %). On peut calculer que la productivité minimale en HFC-143a dans ces conditions est au moins de 200 g.1⁻¹.h⁻¹.

La comparaison des résultats rapportés dans les exemples 1 et 2 indique que la réactivité entre VF2 et HF est beaucoup plus grande en phase liquide qu'en phase gazeuse.

## Revendications

1. Procédé pour la préparation de 1,1,1-trifluoroéthane par réaction de fluorure de vinylidène avec du fluorure d'hydrogène en phase liquide, caractérisé en ce qu'on réalise la réaction en absence de catalyseur.

2. Procédé selon la revendication 1, dans lequel la réaction est réalisée à une température de - 50 à + 120 °C.

3. Procédé selon la revendication 2, dans lequel la réaction est réalisée à une température de - 30 à + 100 °C.

4. Procédé selon une quelconque des revendications 1 à 3, dans lequel on met le fluorure d'hydrogène et le fluorure de vinylidène en oeuvre dans un rapport molaire fluorure d'hydrogène / fluorure de vinylidène de 1 à 20.

5. Procédé selon une quelconque des revendications 1 à 4, dans lequel la réaction est réalisée sous une pression de 2 à 30 bar.

6. Procédé selon une quelconque des revendications 1 à 5, dans lequel la réaction est réalisée dans un réacteur fonctionnant en continu.

7. Procédé selon la revendication 6, dans lequel on travaille avec un temps de séjour des réactifs dans le réacteur de 1 minute à 2 heures.

## Claims

1. Process for the preparation of 1,1,1-trifluoroethane by reaction of vinylidene fluoride with hydrogen fluoride in the liquid phase, characterized in that the reaction is performed in the absence of catalyst.

2. Process according to Claim 1, in which the reaction is performed at a temperature of from -50 to +120°C.

3. Process according to Claim 2, in which the reaction is performed at a temperature of from -30 to +100°C.

4. Process according to any one of Claims 1 to 3, in which the hydrogen fluoride and the vinylidene fluoride are used in a hydrogen fluoride/vinylidene fluoride molar ratio of from 1 to 20.

5. Process according to any one of Claims 1 to 4, in which the reaction is performed at a pressure of from 2 to 30 bar.

6. Process according to any one of Claims 1 to 5, in which the reaction is performed in a reactor working in a continuous manner.

7. Process according to Claim 6, in which the process operates with a residence time of the reactants in the reactor of from 1 minute to 2 hours.

## Patentansprüche

1. Verfahren zur Herstellung von 1,1,1-Trifluorethan durch Reaktion von Vinylidenfluorid mit Fluorwasserstoff in flüssiger Phase, dadurch gekennzeichnet, daß man die Reaktion in Abwesenheit von Katalysator durchführt.

2. Verfahren gemäß Anspruch 1, bei dem die Reaktion bei einer Temperatur von -50 bis +120 °C durchgeführt wird.

3. Verfahren gemäß Anspruch 2, bei dem die Reaktion bei einer Temperatur von -30 bis +100 °C durchgeführt wird.

4. Verfahren gemaß einem der Ansprüche 1 bis 3, bei dem man Fluorwasserstoff und Vinylidenfluorid in einem Molverhältnis Fluorwasserstoff/Vinylidenfluorid von 1 bis 20 einsetzt.

5. Verfahren gemäß einem der Ansprüche 1 bis 4, bei dem die Reaktion unter einem Druck von 2 bis 30 bar durchgeführt wird.

6. Verfahren gemäß einem der Ansprüche 1 bis 5, bei dem die Reaktion in einem Reaktor mit kontinuierlichem Betrieb durchgeführt wird.

7. Verfahren gemäß Anspruch 6, bei dem man mit einer Verweilzeit der Reagentien in dem Reaktor von 1 Minute bis 2 Stunden arbeitet.
